Europäisches Patentamt

European Patent Office

Office européen des brevets

(11) Publication number: **0 319 759 B1**

## EUROPEAN PATENT SPECIFICATION

(45) Date of publication of patent specification: **07.01.93**  (51) Int. Cl.⁵: **C12N 15/10**

(21) Application number: **88119339.5**

(22) Date of filing: **21.11.88**

(54) Method for mutagenesis by oligonucleotide-directed repair of a strand break.

(30) Priority: **11.12.87 US 132089**

(43) Date of publication of application:
**14.06.89 Bulletin 89/24**

(45) Publication of the grant of the patent:
**07.01.93 Bulletin 93/01**

(84) Designated Contracting States:
**DE FR GB**

(56) References cited:
**EP-A- 253 193**
**EP-A- 0 247 647**
**US-A- 4 293 652**

**PROCEEDINGS OF THE NATIONAL ACADEMY OF SCIENCES OF THE USA, vol. 83, October 1986, pages 7177-7181; W. MANDECKI: "Oligonucleotide-directed double-strand break repair in plasmids of Escherichia coli: A method for site-specific mutagenesis"**

**DNA, vol. 7, no. 8, 1988, pages 571-577, Mary Ann Liebert, Inc., Publishers; M.A. HAYDEN et al.: "Gene synthesis by serial clonong of oligonucleotides"**

(73) Proprietor: **ABBOTT LABORATORIES**

**Abbott Park, Illinois 60064(US)**

(72) Inventor: **Mandecki, Wlodzimierz**
**516 Hemlock Lane**
**Libertyville Illinois 60048(US)**
Inventor: **Hayden, Mark Allan**
**80 Commonwealth Court**
**Vernon Hills Illinois 60061(US)**

(74) Representative: **Modiano, Guido et al**
**Modiano & Associati S.r.l. Baaderstrasse 3**
**W-8000 München 5(DE)**

**Description**

Background

This application is a continuation-in-part of application no. 883,242 entitled METHOD FOR MUTAGENESIS BY OLIGONUCLEOTIDE-DIRECTED REPAIR OF A STRAND BREAK filed by Wlodzimierz Mandecki on July 8, 1986, now abandoned.

The present invention relates in general to methods for site-specific mutagenesis and for repair of DNA strand breaks. In particular, the present invention relates to methods for site-specific mutagenesis or DNA break repair wherein an oligonucleotide bearing a desired mutation is employed.

Site-specific mutagenesis is a term which is applied to a large set of in vitro methods [reviewed in Botstein et al., Science, 229 1193-1201 (1985)] which set is generally described as including two classes of procedures: mutagenesis by primer extension and mutagenesis by a gene fragment replacement. In vivo, techniques involving homologous recombination allow targetting of specific sites for mutations.

Primer extension mutagenesis involves hybridization of a mutation-bearing oligonucleotide primer with single-stranded DNA which is the target of mutagenesis. The target DNA serves as a template for the in vitro enzymatic extension of the primer into a complete second strand. The resulting heterologous double-stranded DNA is introduced by transformation into E. coli. A fraction of clones resulting from this transformation carry the mutation.

Gene fragment replacement mutagenesis involves the restriction enzyme digestion in vitro of a double-stranded vector on the outside of a targeted region. Double-stranded synthetic DNA containing a mutation is ligated into the vector at the restriction site.

The primer extension method was initially applied to the site-specific mutagenesis of the single-stranded bacteriophage o174 [Hutchinson, J. Biol. Chem., 253, 6551 1978); Razin et al., Proc. Natl. Acad. Sci. (USA), 75, 4268 (1978)]. A number of protocols allow application of the method to any gene cloned onto a circular vector. The requirement for single-stranded target DNA may be satisfied by: (i) the use of an M13 single-stranded vector [Zoller et al., Nucleic Acids Res., 10, 6487-6500 (1982)]; (ii) the use of a plasmid vector with the origin of replication from M13, which leads to packaging of single-stranded plasmid into virus particles after superinfection with M13 [Dente et al., Nucleic Acids Res., 11, 1645 (1983); Zagursky et al., Gene 27, 183 (1984); and Levinson et al., J. Mol. Acpl. Genet., 2, 507 1984)]; (iii) the enzymatic degradation of a part of one strand of a duplex plasmid with an exonuclease [Wallace et al., Science, 209, 1396 (1980)]; (iv) the creation of a single-stranded gap by forming a heteroduplex molecule [Kramer et al., Nucleic Acids Res., 12, 9441 (1984)]; or (v) the direct annealing of the oligonucleotide to duplex DNA to form a D-loop [Schold et al., DNA, 3, 469 (1984)].

The mutagenesis efficiency of the primer extension methods may be increased by using two primers to initiate DNA synthesis at two sites [Norris et al., Nucleic Acids Res., 11, 5103 (1983); and Zoller et al., DNA, 3, 479 (1984)], or by using a DNA template containing several uracil residues in place of thymine [Kunkel, Proc. Natl Acad. Sci. (USA), 82 488-492 (1985)].

Synthetic DNA to be used for mutagenesis by gene fragment replacement may consist of two annealed oligonucleotides having ends which match ends created by restriction enzymes in vector DNA. Alternatively, mutants may be obtained via the construction of longer gene fragments composed of four or more oligonucleotides, or even whole synthetic genes, [Caruthers et al., "Promoters: Structure and Function", eds. Rodriguez et al., Praeger Scientific, New York, pp. 432-451 (1982)]. Specialized applications of the method include: (i) linker scanner mutagenesis for introducing clusters of point mutations at discrete locations [McKnight et al., Science 217, 316-324 (1982)]; (ii) two codon insertion mutagenesis with the use of selectable marker [Barany, Proc. Natl. Acad. Sci. (USA), 82 4202-4206 (1982)]; and (iii) "bandaid" mutagenesis which relies on ligating a single oligonucleotide into a double-stranded gap created by cleavage with two restriction endonucleases which form a single-stranded 3′ end and a single-stranded 5′ end respectively complementary to either end of the oligonucleotide [Mural et al., DNA, 5, 84 (1986)].

The above methods are universal and permit introduction of defined point mutations, insertions and deletions in virtually any desired location on a cloned DNA. Therefore, site-directed mutagenesis is widely used to probe the structure-function relationship in DNA, RNA and protein, to study the interaction of these biological macromolecules and to elucidate pathways of genetic regulation. The broad applicability of site-directed mutagenesis warrants interest in novel mutagenesis methods, especially in methods which reduce the time and effort currently required to obtain a mutant.

Although the phenomena of primer extension and gene fragment replacement are well explained, the phenomenon of DNA double-strand break repair in E. coli is not well understood. Plasmid DNA linearized at a unique restriction site transforms E. coli, although with much lower efficiency than the corresponding covalently closed circular DNA form [Thompson et al., Mol. Gen. Genet., 169, 49-57 (1979); and Conley et al., Mol. Gen. Genet., 194, 211-218 (1984)]. Although transformants containing plasmids bearing deletions or rearrangements are frequent, transformants carrying the native plasmid molecules are also obtained. Simple intracellular reannealing and ligation of the two cohesive termini of a linear molecule is thought not to be the predominant mechanism of plasmid establishment, since transformation with linearized plasmid DNA is reduced 10 to 40-fold in recA, recBC, or recF backgrounds [Conley et al., Mol. Gen. Genet., 194, 211-218 (1984)].

In a variation of a process called homologous recombination, linearized chimeric plasmid DNA may transform cells containing a resident plasmid which is homologous with a portion of the chimeric plasmid DNA in the process called "marker rescue transformation". Marker rescue transformation is recE-dependent, requires that the chimeric and resident plasmids have homologous sequences and that homologous sequences flank any nonhomologous segment to be "rescued," varies in efficiency with the position of the linearizing cut in the chimeric plasmid DNA, and is a first order reaction with respect to DNA concentration [Contente et al., Plasmid, 2, 555-571 (1979)].

In yeast, restriction enzyme digestion within a region of a circular hybrid plasmid, which region is homologous to chromosomal DNA, greatly enhances the efficiency of integration of plasmid DNA into chromosomal DNA over the efficiency obtainable with circular plasmid DNA [Orr-Weaver et al., Proc. Natl. Acad. Sci. (USA), 78, 6354-6358 (1981)]. By locating the cut site within the region of the plasmid DNA which is homologous to a site on the chromosome, integration of the plasmid may be targeted to the chromosomal site [Orr-Weaver et al., supra]. The efficiency of transformation is highly dependent on plasmid concentration [Orr-Weaver et al., supra]. The integration of linear molecules having a deletion within the region of homology is accompanied by repair of the missing information using the homologous region of the chromosome as a template, and the integration process may require the same RAD52 gene product required for double-strand break repair and for gene conversion [Orr-Weaver et al., supra]. However, the insertion of a desired sequence leads to the formation of a direct repeat of the homologous sequence in the chomosome and to the undesirable insertion of vector sequences into the chromosome [Orr-Weaver et al., Methods Enzymol., 101 228-245 (1983)]. Moreover, although subsequent recombination of the duplicated sequences may eliminate the undesired duplicate and vector sequences, this elimination occurs at a very low rate [Scherer et al., Proc. Natl. Acad. Sci. (USA), 76 4951-4955 (1979); and Scherer et al., Science, 209, 1380-1384 (1980)]. For example, excision of undesired vector sequences was observed in seven out of 900 isolates after 10 generations and in only three of those isolates had wild type gene been deleted to successfully complete replacement by a single copy of an inserted mutant gene [Scherer, Proc. Natl. Acad. Sci. (USA), supra].

The homologous recombination technique of Orr-Weaver et al., Proc. Natl. Acad. Sci. (USA), supra, may be employed to target gene insertion into mammalian chromosomes, for example to target the insertion of a plasmid containing a beta-globin deletion analog at the beta-globin locus [Smithies et al., Nature, 317 230-234 (1986). However, as in yeast, the insertion of a desired sequence leads to the formation of a direct repeat of the homologous sequence in the chromosome and to the undesirable insertion of vector sequences therein [Smithies et al., supra]. The low frequency of homologous recombination and the low frequency with which excision events occur limits the potential use of the technique in gene therapy [Maniatis, Nature, 317, 205-206 (1986)].

In B. subtilis, homologous recombination may be employed to insert nonhomologous sequences linked to flanking sequences homologous to the chromosome (and thus to clone a gene if the nonhomologous sequence is a gene) or to delete chromosomal sequences when the flanking sequences are nonadjacent (upon the occurrence of a double crossover recombination event) [Niaudet et al., Gene, 19, 277-284 (1982); and Niaudet et al., J. Bacteriol., 163, 111-120 (1985)]. Introduction of plasmid-borne sequences which are homologous to sequences within a cell but for alterations (i.e., some types of deletions, additions, or inversions) may be cured to complete homology after transformation into the cell [Iglesias et al., Mol. Gen. Genet., 184, 405-409 (1981)]. Nevertheless, the usefulness of these techniques is subject to limitations similar to those present in homologous recombination in yeast.

Proc. Natl. Acad. Sci. USA 83, 7177-81 (1986) and EP-A-253193, which is a conflicting European application for the designated contracting states DE and FR in accordance with Article 54(3) and (4) EPC, disclose a method of oligonucleotide directed repair of double stranded DNA breaks to obtain deletion mutants.

US-A-4293652 describes a method for sequential gene assembly using a double stranded synthetic oligonucleotide and an in vitro ligation step.

Summary of the Invention

The present invention provides a method for insertion or substitution of nucleotides by a process which includes the step of cocultivating within an appropriate host cell both linear target DNA having a break and an oligonucleotide having a nucleotide sequence complementary to a strand of the target DNA on both sides of the break and spanning the break. As employed herein, the term "break" as applied to a segment of DNA, either single- or double-stranded, refers to a separation between ends, including both blunt and cohesive ends. Thus, the term "break" is applied to the separation between ends of single- or double-stranded linear DNA but not to a nick or to a gap in only one strand of double-stranded DNA.

According to the present invention the oligonucleotide includes a first portion (a "portion" being one or more nucleotides in length) completely complementary to the nucleotide sequence of a first region (a "region" being one or more nucleotides in length) of the strand, a second portion completely complementary to the nucleotide sequence of a second region of the strand, and a third portion which is between the first and second portions and which is not complementary to nucleotide sequence of the strand between the first and second regions. Thus, "spanning the break" means bringing the two ends of the target into physical proximity.

The method also includes the step of maintaining said host cell under appropriate conditions, especially conditions favorable for strand break repair, and for a period sufficient to permit incorporation into the target DNA of a third region complementary to the third portion between the first region and the second region. "Incorporation" in the sense in which it is used herein refers to any process by which the desired result is obtained, particularly including filling and ligation and including but not limited to the process of repair.

The cocultivating step of the method for site-directed mutagenesis according to the present invention preferably includes the step of exposing the oligonucleotide to double-stranded target DNA in vitro and more preferably includes the step of cointroducing the oligonucleotide and the target DNA into the host cell. "Introduction" may be by any suitable means, including but not limited to injection, CaCl$_2$ transformaticn, electroporation, and cell fusion or fusion with liposomes. A cocultivating step further including the step of denaturing the target DNA is presently preferred.

In the site-directed mutagenesis method according to the present invention, DNA on a vector (autonomously replicating DNA or RNA), preferably a plasmid, may be employed as the target. Although a plasmid is preferred, a viral vector such as a bacteriophage may also be employed as convenient.

The exposing step. may involve transforming the host cell with the oligonucleotide, transforming the host cell with the target DNA, and, preferably, cotransforming (i.e., transforming at about the same time and especially where the oligonucleotide and the target DNA are included in the same transformation medium) the host cell with the target DNA and with the oligonucleotide. The exposing step may include the step of providing an oligonucleotide having a third portion the most medial nucleotide of which is adjacent a nucleotide of the second portion which is no more than about 26 nucleotides distant from the break along the nucleotide sequence of the strand of the target DNA.

A method for obtaining a deletion of nucleotides according to the present invention includes the step of cocultivating within an appropriate host cell both target DNA having a break, and an oligonucleotide. The sequence of the oligonucleotide is complementary to regions of a strand of the target DNA on both sides of the break and spanning the break, and includes: a first portion completely complementary to the nucleotide sequence of a first region of the strand, a second portion completely complementary to the nucleotide sequence of a second region of the strand and immediately adjacent to the first portion, wherein the target DNA comprises a third region between the first and second regions; and maintaining said host cell under appropriate conditions and for a period sufficient to permit incorporation into the target DNA of a third region complementary to the third portion between the first region and the second region. The method may also have a cocultivating step including the steps of exposing the oligonucleotide to double-stranded target DNA and denaturing the target DNA. The method may have the additional step of providing about a 1000-fold excess of the oligonucleotide as compared to target DNA.

The present invention also provides a method for DNA double-strand break repair. Within an appropriate host cell, target DNA having a double-strand break is cocultivated with an oligonucleotide having a nucleotide sequence complementary to a strand or the target DNA on both sides of the break wherein the oligonucleotide includes a first portion completely complementary to the nucleotide sequence of a first region of the strand and located on a first side of the double-strand break, and a second portion completely complementary to the nucleotide sequence of a second region of the strand and located on a second side of the double-strand break. The host cell is maintained under appropriate conditions and for a period sufficient to permit repair of the double-strand break.

In the method for DNA double-strand break repair according to the present invention, an oligonucleotide may be provided which includes a third portion which is located between the first and second portions and which is not complementary to the strand between the first and the second regions.

The present invention further provides a method for repair of a strand break in single-stranded DNA which involves cocultivating within an appropriate host cell both target DNA having a single-strand break and an oligonucleotide having a nucleotide sequence complementary to a strand of the target DNA on both sides of the break. The oligonucleotide has a first portion completely complementary to the nucleot;de sequence of a first region of the strand and located on a first side of the single-strand break, and a second portion completely complementary to the nucleotide sequence of a second region of the strand and located on a second side of the single-strand break. The host cell is maintained in under appropriate conditions and for a period sufficient to permit repair of the single-strand break. The method for DNA single-strand break repair may include a step of providing an oligonucleotide comprising a third portion which is located between the first and second portions and which is not complementary to the strand between the first and the second regions. Thus, the method may be used for site-directed mutagenesis.

According to the present invention, a method for performing gene therapy to obtain a desired genetic modification includes the step of providing cells having a break in target DNA corresponding to the gene to be treated. It also involves the step of introducing into the cells to be treated an oligonucleotide having a nucleotide sequence complementary to a strand of the target DNA on both sides of the break and wherein the oligonucleotide includes a first portion completely complementary to the nucleotide sequence of a first region of the strand, a second portion completely complementary to the nucleotide sequence of a second region of the strand, and a third portion which is between the first and second portions and which is not complementary to nucleotide sequence of the strand between the first and second regions. The host cell is maintained under appropriate conditions and for a period sufficient to permit incorporation of a third region complementary to the third portion between the first region and the second region. The maintaining step may include the step of selecting for cells exhibiting the desired genetic modification.

In order to create a break in the DNA, the cells may be exposed to radiation under conditions suitable for creating a break in DNA by the application of radiation, or by applying a drug to the cells under conditions suitable for creating a break in the DNA by the application of the drug. Most preferably, however, a sequence-specific DNA cleaving molecule having a specificity for the portion of the target DNA which is to be modified is introduced into the cells.

A method for cloning a segment of DNA according to the present invention includes the step of cocultivating within an appropriate host cell vector DNA having a break and linear target DNA to be cloned. Also cocultivated are a first and a second oligonucleotide. The first oligonucleotide includes a first portion completely complementary to the nucleotide sequence of a first region of a strand of the vector DNA, the first region being located on a first side of the break, and a second portion completely complementary to the nucleotide sequence of a first region of a strand of the target DNA, the second region being located proximal to a first end of the target DNA and distal to a second end of the target DNA. The second oligonucleotide has a first portion completely complementary to the nucleotide sequence of a second region of a strand of the vector DNA, the second region being located on a second side of the break, and a second portion completely complementary to the nucleotide sequence of a second region of a strand of the target DNA, said second region being located proximal to the second end of the target DNA and distal to the first end. The host cell is maintained under appropriate conditions and for a period sufficient to permit repair of a break.

Brief Description of the Drawings

Fig. 1 illustrates the sequence of the multicloning site of a vector before and after mutagenesis according to the present invention as well as the homologous sequence of a plasmid resulting from a second mutagenesis according to the present invention;

Fig. 2 is a schematic illustration of the mutagenized vector of Fig. 1 and of the resulting plasmid showing the site of mutagenesis aligned with the mutagenizing oligonucleotide;

Fig. 3 illustrates the sequences of oligonucleotides of various lengths employed according to the present invention;

Fig. 4 is a graphic depiction of the relationship between length of the oligonucleotide employed according to the present invention and both the efficiency of mutagenesis and the number of mutant colonies produced;

Fig. 5 is a graphic depiction of the relationship between the amount of linearized plasmid DNA employed according to the present invention and both the efficiency of mutagenesis and the number of mutant colonies obtained;

Fig. 6 is a graphic depiction of the relationship between the concentration of oligonucleotide used, and both the efficiency of mutagenesis and the number of mutant colonies obtained;

Fig. 7 is a schematic illustration of the sequences of oligonucleotides overlapping the cleavage site and having mutations at various distances from the cleavage site of a target plasmid according to the present invention;

Fig. 8 is a graphic depiction of the relationship between the distance between the cleavage and mutation sites (holding the location of the cleavage site constant), and both the efficiency of mutagenesis and the number of mutant colonies obtained;

Fig. 9 schematically illustrates the sequence of an oligonucleotide aligned with a homologous sequence in a plasmid cleaved at restriction sites as shown; and

Fig. 10 is a graphic depiction of the relationship between the distance between the cleavage and mutation sites (holding the location of the mutation site constant), and both the efficiency of mutagenesis and the number of mutant colonies obtained.

Fig. 11 gives a graphic depiction of the dependence between the length of the insert and both the efficiency of mutagenesis and the number of mutant colonies obtained.

Fig. 12 shows the sequence of the fragment of the p41 gene of HIV, which was constructed using bridge mutagenesis.

Fig. 13 is a graphic depiction of the deletion of a gene sequence using bridge mutagenesis.

Fig. 14 is a graphic depiction of the sequential insertion of gene fragments into a gene using bridge mutagenesis;

Fig. 15 is the amino acid sequence of human anaphylatoxin complement C3a with a nucleic acid sequence therefor;

Fig. 16 shows a Bsm I recognition sequence and cutting pattern;

Fig. 17 is a graphic depiction of the construction of pMH1.

Fig. 18 is a graphic depiction of X-Gal color screening of cells of the present invention;

Fig. 19 is a graphic depiction of three oligodigodeoxyribonucleotides, each having a portion corresponding to a DNA sequence encoding for a portion of C3a.

## Detailed Description

According to the present invention, an "oligonucleotide", in particular an oligodeoxyribonucleotide having a sequence derived from DNA sequences on the two sides of a DNA double-strand break (i.e. spanning the break) may direct the repair of the break. As a result, the efficiency of transformation of E. coli with denatured linearized plasmid DNA may be increased by orders of magnitude in the presence of the oligonucleotide. If the sequence of the oligonucleotide contains a mutation, the mutation may be incorporated into the repaired plasmid DNA.

The mutagenesis protocol according to the present invention includes a minimal number of steps. Heat-denatured linearized plasmid DNA and the oligonucleotide are introduced by transformation into E. coli, and transformants screened for the mutation. Unlike "bandaid" mutagenesis, the present invention does not require an in vitro, enzymatic reaction (such as ligation), and the present invention is not restricted in its use to DNA having a particular type of end (such as the 3′- and 5′-protruding ends required for "bandaid" mutagenesis).

The transformants obtained are screened for the presence of the mutation using methods such as restriction enzyme digestion, colony hybridization, or DNA sequencing.

The procedure for mutagenesis according to the present invention is applicable in situations where plasmid DNA may be linearized in the immediate vicinity of the site to be mutagenized. Where possible, linearization may be accomplished by cleaving DNA at a unique restriction site within a few nucleotides of the residue to be mutated. If a unique restriction site is not available, the following alternative methods of linearization might be used: (i) partial digestion with restriction enzymes which cleave at more than one site; (ii) reconstructing a linear plasmid by the ligation of DNA fragments obtained with other restriction enzymes; or (iii) random cleavage of DNA under mild conditions (e.g by DNase digestion, or sonication).

The method is particularly well suited to such applications as joining of non-matching ends of plasmid DNA (an alternative to in vitro enzymatic manipulations), destruction and/or introduction of a restriction site, random localized mutagenesis with oligonucleotide probes with degeneracies, or generation of defined deletions following DNA exonuclease digestion. The method has been observed to be successful in rec⁻ host cells but not where the target DNA had no break spanned by the oligonucleotide (e.g. where the target DNA was covalently closed circular DNA).

The method according to the present invention is more specifically described in the following Examples. In Example 1, the method according to the present invention is employed to delete a nucleotide from the pUC9 plasmid. Example 2 illustrates the use of the present invention to insert a nucleotide into the plasmid resulting from the manipulation of Example 1. The relationship between oligonucleotide length and the efficiency of mutagenesis, defined herein as the ratio (percent) of the number of mutants to the total number of transformants obtained, is examined in Example 3. In Example 4, the relationship in the practice of the present invention between the relative amounts of plasmid DNA and oligonucleotide and the efficiency of mutagenesis is explored.

Example 5 illustrates the importance of selecting an oligonucleotide which spans the cleavage site. In Example 6, the efficiency of mutagenesis is determined for variations in the position of the mutation site with respect to a linearization site at a constant position. In Example 7, the relative position of the linearization site is varied with respect to the constant position of a mutation in order to determine the effect on the efficiency of mutagenesis. In Example 8, the relationship between the condition or the linearized DNA (i.e. denatured versus non-denatured) is examined.

In Example 9, the universal applicability of the present invention is illustrated through the use of a variety of plasmids, host strains, restriction enzymes, genes and mutations. Example 10 illustrates the applicability of the present invention to mutagenesis of chromosomal DNA in situ. Example 11 describes the use of the present invention in "gene therapy". In Example 12, the present invention is applied to the cloning of genes.

Example 1

A plasmid system having three properties was sought for a quantitative characterization of the mutagenesis method. The properties were: (i) the presence of a cluster of unique restriction sites for cleaving plasmid DNA; (ii) the plasmid's applicability for color screening of mutant colonies; and (iii) the accuracy of the mutant screening method. The pUC9 plasmid [Vieira et al., Gene 19, 259-268 (1982)] satisfied the first two criteria, because of its multicloning site and because of its indicator gene (a fragment of the lacZ gene encoding the enzyme beta-galactosidase which digests indolyl-beta-D-galactoside present in the medium of cells transformed with the vector to produce a blue pigment). However, the third criterion was not met by pUC9 because both mutations (such as nonsense or frameshift mutations) of the multicloning site within the lacZ gene and cells carrying rearranged plasmids give rise to colonies of the same type (white, on a background or wild-type blue colonies). This made a precise quantitation of mutant colonies difficult.

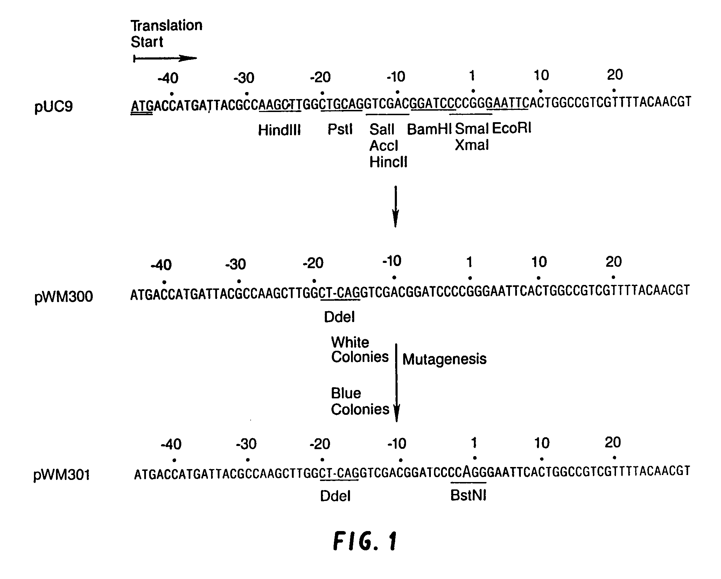

The third requirement was met by constructing a derivative of the pUC9 plasmid carrying a frameshift mutation in the multicloning site. The mutant plasmid, pWM300, was generated using the mutagenesis method according to the present invention, as shown in Fig. 1.

In Fig. 1 sequences of the multicloning site of pUC9 and two derivatized plasmids are shown. Plasmid pWM300, a frameshift mutant of pUC9, was constructed by the DNA double-strand break repair according to the present invention. The oligonucleotide used for mutagenesis encompassed residues -45 to 14 of pWM300 (as numbered in Fig. 1).

Employing the method according to the present invention, pUC9 plasmid DNA was linearized with the PstI restriction enzyme. The completion of the reaction was confirmed by agarose gel electrophoresis. Uncleaved circular plasmid molecules, if used in the procedure, increase the background of the wild-type clones. The plasmid DNA and oligonucleotide were then combined in a denaturation mixture. The denaturation mixture consisted of 50 ng of linearized plasmid DNA and 20 pmol of oligonucleotide in a buffer composed of 10 mM KCl, 5 mM Tris-HCl pH 8.0, 5mM MgS04, 0.5 mM dithiothreitol in a total volume of 30 ul in a 1.5 ml Eppendorf tube.

The mixture was incubated at 100°C for 3 min. (in a boiling water bath), the tube was then removed to room temperature for 5 min., the contents of the tube were transferred to 200 ul of chilled competent cells prepared by CaCl₂ treatment [according to Mandel et al., J. Mol. Biol. 53, 159-162 (1970)] and gently mixed. The cells and DNA were incubated on ice for 5 min. and then subjected to a 3 min. heat shock at 37°C. After adding 2 ml of 2 x YT medium [prepared according to Miller, "Experiments in Molecular Genetics", Cold Spring Harbor Laboratory, Cold Spring Harbor, p. 17 (1972)], the cells were incubated at 37°C for 1 hr, and then plated on LB plates [prepared according to Miller, "Experiments in Molecular Genetics", Cold Spring Harbor Laboratory, Cold Spring Harbor, NY, p. 433 (1972)] containing 100 ug/ml ampicillin [GIBCO, Grand Island Biological Company, Grand Island, New York] and 20 ug/ml of 5-bromo-4-chloro-3-indolyl-$\beta$-D-galactoside (Sigma Chemical Company, St. Louis, Missouri), as appropriate.

Methods for large scale plasmid isolation, minipreparations of plasmid DNA and restriction enzyme digestions were performed as described in [Davis et al., "Advanced Bacterial Genetics", Cold Spring Harbor Laboratory, Cold Spring Harbor, NY, pp. 116-117 (1980)]. The primer extension method was applied for DNA sequencing, using denatured plasmid DNA as a template according to Chen et al., DNA 4, 165-170 (1985).

Example 2

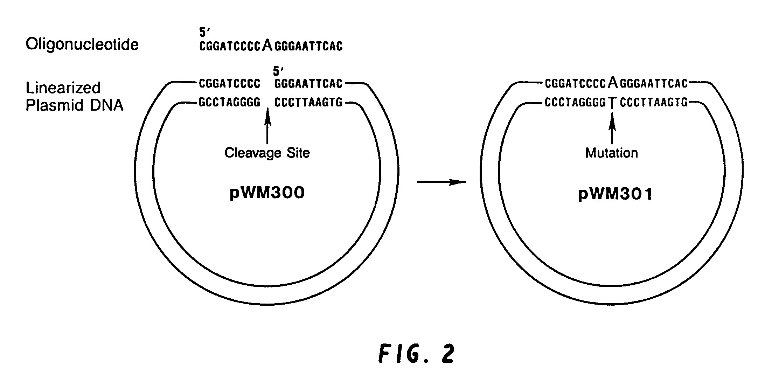

Plasmid pWM300 was employed as a substrate for further mutagenesis. As illustrated in Fig. 2, plasmid pWM300 was simultaneously linearized and blunt-ended by cleavage with the enzyme SmaI. Upon mutagenesis with the oligonucleotide shown in Fig. 2 according to the mutagenesis procedure set forth in Example 1, plasmid pWM301 was created. Thus the coding sequence within the multicloning site was brought in frame to allow for the color screening of transformants. In addition, a BstNI site was generated, which allowed confirmation of the presence of the mutation by restriction analysis. The position of the mutation is indicated by an oversize letter.

As set forth in the following Examples, mutations introduced into the pWM300 system to investigate the mutagenesis method according to the present invention were typically insertions of one residue which restored the reading frame of the lacZ gene fragment. In this system, the mutants formed blue colonies, while cells containing only the parent plasmid (pWM300) and/or rearranged plasmids, gave rise to white colonies.

All insertions introduced into plasmid pWM300 were designed such that they would destroy a unique restriction site within the multicloning site, and at the same time create a new restriction site. This feature allowed for an additional confirmation of the mutagenesis event by restriction digestion of plasmid DNA.

Example 3

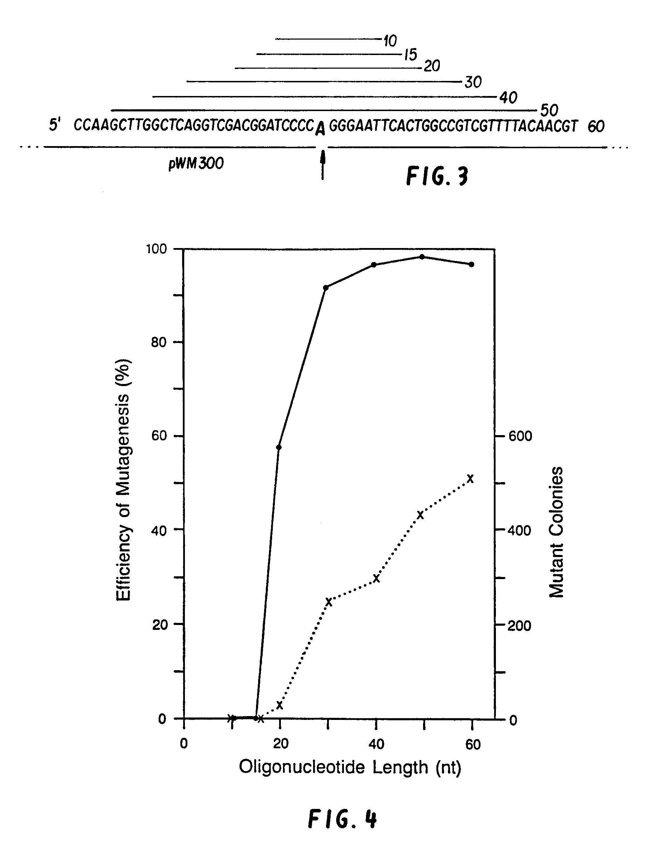

A set of oligonucleotides 10 to 60 residues long was synthesized in order to determine the effect of the length of the oligonucleotide upon the efficiency of mutagenesis. Oligonucleotides were synthesized by the phosphoramidite method [Caruthers, Science 230, 281-285 (1985)] on a model 380A synthesizer available from Applied Biosystems (Foster City, California) and were purified by polyacrylamide gel electrophoresis under denaturing conditions. The sequences of oligonucleotides were homologous to the segment of plasmid pWM300 (as shown in Fig. 1) and were symmetrical with respect to the SmaI site of pWM300, spanning this site. The fragments carried an insertion of one adenine residue into the SmaI site as indicated in Fig. 3. Plasmid pWM300 linearized by digestion with SmaI restriction enzyme was used for mutagenesis.

Fig. 3 and Fig. 4 illustrate the role of the length of oligonucleotide in mutagenesis. The sequence of the longest oligonucleotide used (a 60-mer) is given in Fig. 3. Sequences of other oligonucleotides were subsequences of the 60-mer, as indicated by horizontal bars. The lengths of oligonucleotides are given at the right end of each oligomer. The mutation introduced is indicated by the largest letter in the 60-mer. In the solid line represented plasmid pWM300, the SmaI cleavage site is representing by an arrow at a break in the line. Plasmid pWM300 (50 ng) was linearized at the SmaI site and a 20 pmol of each of the oligonucleotides shown in Fig. 3 was used for mutagenesis according to the procedure of Example 1.

In figure 4, the results of the experiment are presented. The solid line is a graph of the efficiency of mutagenesis; the dotted line is a graph of the number of mutant colonies per plate. Twenty clones that formed blue colonies were tested by digestions with endonuclease BstNI (BstNI was a newly created restricton site) to confirm the presence of the desired mutation. All 20 clones showed the expected digestion pattern as demonstrated by agarose gel electrophoresis. The DNA sequence of the multicloning site was obtained as described above for two clones. The sequence was as expected for the mutant.

A high efficiency of mutagenesis of over 90% was observed using oligonucleotides of 30 or more residues in length as shown in Fig. 4, and the maximal efficiency was 98% for the 50 residue long oligonucleotide. The 20-residue long oligonucleotide resulted in a half-maximal efficiency, while no mutants were observed using either the 10-mer or the 15-mer. Although the efficiency of mutagenesis seemed to be reaching a plateau using oligonucleotides 40 or more residues in length, the number of mutant colonies continued to increase with the length of DNA. Thus, in order to assure a good efficiency of mutagenesis, an oligonucleotide for use in the method according to the present invention is preferably at least 20 to 30 residues in length, and more preferably longer.

Example 4

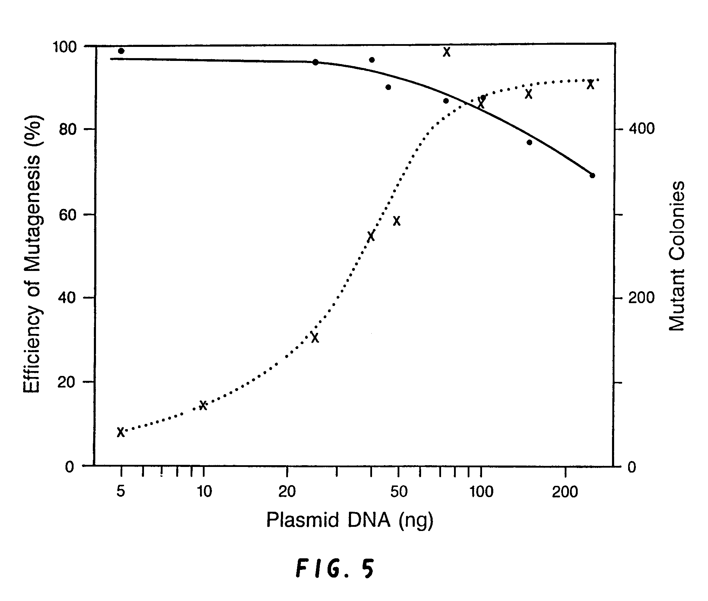

The relationship between the efficiency of mutagenesis according to the procedure of Example 1 and the amount of the linearized plasmid DNA is shown in Fig. 5 in which the solid line illustrates efficiency of mutagenesis; and the dotted line is a graph of the number of mutant colonies per plate.

The pWM300 plasmid linearized at the SmaI site in the amount indicated in Fig. 5 and 20 pmol of the 60-mer depicted in Fig. 3 were used. Titrations with plasmid DNA and oligonucleotide were done to optimize amounts of DNA used for mutagenesis.

As shown in Fig. 5, when the concentration of oligonucleotide was kept constant (20 pmol of the 60-mer per reaction), the efficiency of mutagenesis decreased if over 50 ng of plasmid DNA was used. However, the number of mutants generated increased with the amount of plasmid DNA in the reaction to reach a plateau at about 100 ng DNA. It is preferred, therefore, that about 50 ng of linearized plasmid DNA be used for mutagenesis.

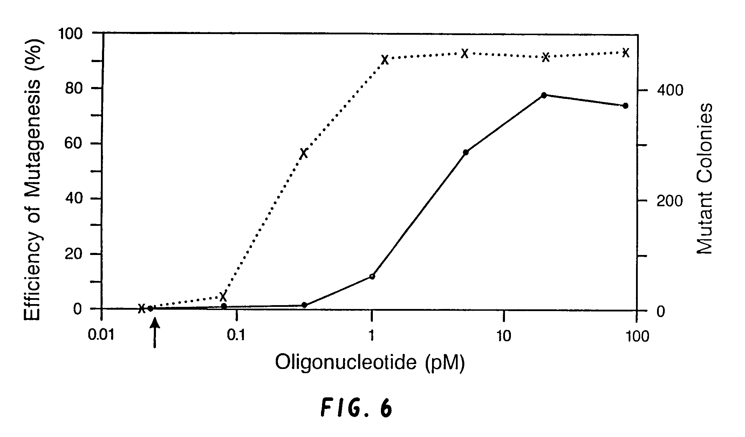

A large molar excess of oligonucleotide over plasmid DNA is important fcr mutagenesis, as is evident from Fig. 6. In Fig. 6, the indicated amount of the 60-mer of Fig. 3 and 50 ng (approximately 20 fM, as indicated by the arrow) of SmaI-cleaved pWM300 were employed in the procedure according to Example 1. In Fig. 6 the solid line is a graph of the efficiency of mutagenesis and the dotted line is a graph of the number of mutant colonies per plate.

As illustrated in Fig. 6, while the efficiency of mutagenesis reached a plateau at a lpm concentration of oligonucleotides, the maximal number of mutant colonies was obtained with 20 pmol of oligonucleotide. This amount corresponded to a 1000-fold molar excess of oligonucleotide.

Example 5

In order to better determine the possible mutant-yielding locations of sequence changes within the oligonucleotide and position of the oligonucleotide with respect to the linearization site several experiments were performed employing the mutagenesis procedure of Example 1.

First, in order to determine whether it is important that the oligonucleotide spans the cleavage site, the plasmid pWM300 was linearized with the NdeI restriction enzyme, which cleaves at a site about 240 bp from the multicloning site. It was then tested whether the 60-mer sequence given in Fig. 3 which sequence spans the SmaI site, could induce mutations in the NdeI-cleaved plasmid.

Among 269 transformants no mutants were observed. Typically, if the SmaI-cleaved pWM300 plasmid is used under the same conditions, over 95% of transformants are mutants. The results demonstrate that to obtain mutants it is important that the oligonucleotide spans the linearization site.

Example 6

A set of oligonucleotides was synthesized to define the dependence of mutagenesis according to the procedure of Example 1 on the distance between a mutation and the linearization site.

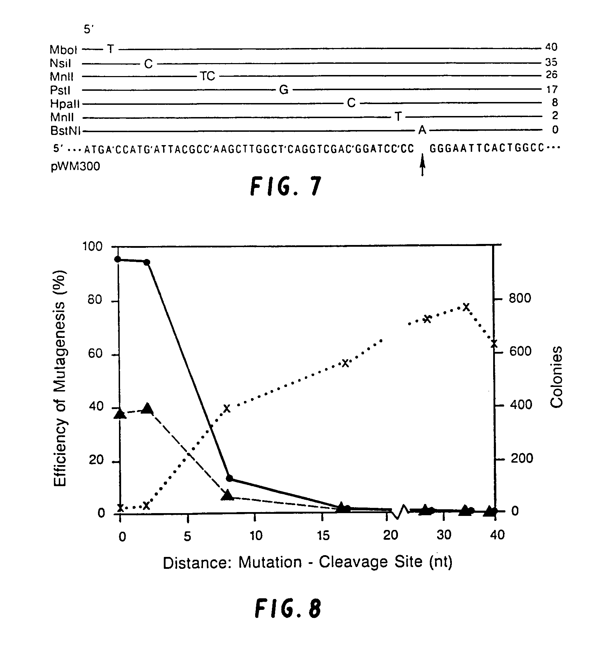

The oligonucleotides were eight asymmetrical 60-mers with insertions of one residue at different positions. The sequences of 60-mers which were used for mutagenesis are shown in Fig. 7 as modifications of the sequence of the multicloning site of plasmid pWM300. In Fig. 7, positions of insertion mutations are indicated on horizontal bars representing oligonucleotides. The exact locations of inserted residues are defined by apostrophes on the pWM300 sequence. The insertions generated new restriction sites, which are given at the left end of the line. The oligonucleotide that introduced the MnlI site contained an insertion of one residue combined with a point mutation (...GCCAAG...-...CCTCAG...). The distance between the mutation and the cleavage site is defined as the length of the spacer DNA between the two. The distances (in nucleotides) are given on the right side of the figure.

The 60-mers were tested for their ability to generate mutations in the pWM300 plasmid linearized at the SmaI site. The results are illustrated in Fig. 8 in which a solid line graphically depicts the efficiency of mutagenesis, a broken line graphically depicts the number of mutant colonies, and a dotted line graphically depicts the number of non-mutant colonies formed by cells carrying the parent or rearranged plasmids. The results showed that the efficiency of mutagenesis results showed that the efficiency of mutagenesis decreased rapidly from 98% to 13% as the distance between the mutation and the cleavage site increased from 0 to 8 base pairs (bp). Half-maximal efficiency was obtained at the distance of about 5 bp.

The efficiency of mutagenesis at the distance of 17 nucleotides (nt) was 1.4% (6 mutants per 422 transformants, confirmed by DNA sequencing). The distance of 26 nt between the mutation and the cleavage site was the maximum that allowed to obtain a mutation, and the efficiency of mutagenesis was 0.2% (2 mutants per 820 transformants, confirmed by DNA sequencing).

In parallel to the decrease in the number of mutants per plate, the number of non-mutant white colonies increased. This strongly suggests that if the mutation is distal to the cleavage site (SmaI), the wild-type sequences around the SmaI recognition sequence on the oligonucleotide can direct the repair and establishment of the plasmid DNA.

Example 7

In an alternative approach to the investigation of Example 6, the position of the linearization site is varied utilizing different restriction enzymes which cleave within the multicloning site.

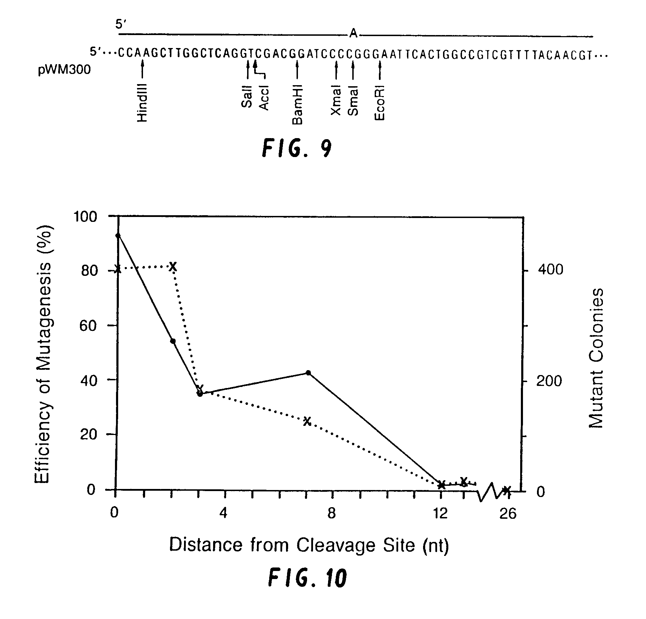

The 60-mer carrying a mutation at the SmaI site (sequence given in Fig. 3) was used to direct the repair according to the mutagenesis procedure of Example 1. The oligonucleotide used was the 60-mer of Fig. 3, and it is indicated with a heavy line in Fig. 9. Linear plasmid DNA was prepared by digesting pWM300 with one of several restriction endonucleases. Their recognition sites are represented by arrows. One plasmid strand with polarity identical to that or the oligonucleotide of Fig. 1 was arbitrarily chosen to assign locations of the endonuclease cleavage sites.

The results of this experiment, as graphically depicted in Fig. 10, indicate the dependence of the efficiency of mutagenesis (solid line) and number of mutant colonies (dotted line) on the distance between a mutation and the linearization site. The distance is defined here as the length of the spacer DNA between the mutation and the endonuclease cleavage site. As shown in Fig. 10, the mutagenesis efficiency decreased to 40% at the distance of 7 bp and 2% at the distance of 13 bp.

There are several geometric factors to consider when designing an oligonucleotide for mutagenesis. Because a 20-mer was the shortest oligonucleotide for which mutagenesis was observed, the overlap between the oligonucleotide and plasmid DNA on each side of the cleavage site has to be at least 10 nt. Mutants were not obtained if the mutation was located less than 10 nt from the end of oligonucleotide (as indicated in Fig. 8 and in an analogous experiment with 40-mers, data not shown). Evidently, to be incorporated into plasmid DNA the mutation should be at least 10 nt from an end of the oligonucleotide used.

Example 8

The relationship between the condition (i.e. denatured versus non-denatured) of the linearized DNA was examined.

When non-denatured, SmaI digested pWM300 plasmid DNA was used for mutagenesis together with the 60-mer (sequence given in Fig. 3) in the procedure of Example 1, only 6 mutants were obtained among a total of 57 transformants. In a parallel experiment, the use of heat-denatured plasmid DNA resulted in 1330 mutants per a total of 1360 transformants.

Thus, for a high efficiency of mutagenesis it is important to denature the linear plasmid DNA before using it for transformation. This is done by incubating a mixture of plasmid DNA and the oligonucleotide at 100°C for 3 min. The use of non-denatured DNA results in a much lower efficiency of mutagenesis.

Example 9

In order to demonstrate that the mutagenesis method according to the present invention is not limited to use with any particular strain, a total of four plasmids, three E. coli strains, three genes and eight restriction enzymes were used in the process of introducing different mutations, as summarized in Table 1. Within the range of ± 10 bp from the cleavage site the mutations were introduced into a plasmid in all attempted cases utilizing the method of mutagenesis described in the Example 1 above.

Four classes of mutations were generated, namely:
insertion of one residue, deletion of one residue, point mutation, and point mutation combined with an insertion of one residue. It should be possible to obtain larger insertions, deletions or multiple point mutations utilizing a single oligonucleotide.

TABLE I

MUTATIONS INTRODUCED

| Plasmid | Strain | Type of Mutation | Gene/site mutated | Cleavage site | Distance mutation-cleavage site (nt) | Length of oligonucleotide used (nt) | Efficiency (%) |
|---|---|---|---|---|---|---|---|
| pN01523 | MC1009 | Insertion of 1 residue | rpsL gene | SmaI | 0 | 60 | 88 (7/8) |
|  | CSH26 | " | " | " | " | " | 92 (12/13) |
| pUC9 | JM83 | Deletion of 1 residue | Multicloning site | PstI | 2 | 60 | 25 (1/4) |
| pWM111 | JM83 | Point mutation | C5a gene | PstI | 3 | 59 | 29 (2/7) |
|  |  | " | " | " | 0 | 59 | 57 (4/7) |
|  |  | " | β-lactamase gene | ScaI | 0 | 60 | 25 (4/12) |
| pWH1300 | JM83 | Insertion of 1 residue, point mutation, combination of both | Multicloning site | SmaI XmaI EcoRI BamHI SalI AccI | 0-26 | 20-60 See text for details | 0.2-98 |

In Table 1 the number of mutants per a total number of transformants screened by DNA restriction analysis is enclosed within parentheses. The constructions were confirmed by DNA sequencing of the relevant region in at least one clone of each class. All procedures were performed as set forth in Example 1.

For use with pN01523 [Dean, Gene 15, 99-102 (1981)] in host strains MC1009 [Casadaban et al., J. Mol. Biol. 138 179-207 (1980) or CSH26 [Miller, "Experiments in Molecular Genetics," Cold Spring Harbor Laboratory, Cold Spring Harbor, NY, p. 17 (1972)], the nucleotide sequence of the oligonucleotide was the same as the nucleotide sequence from residue 371 to residue 429 of the non-coding strand of the rpsL gene [Post et al., J. Biol. Chem. 255 4660-4666 (1980)]. The oligonucleotide contained a mutation that led to the insertion of an A/T base pair between residues 401 and 402, creating a BstNI site.

11

The construction of the oligonucleotide used for pUC9 [Vieira et al., Gene 19, 259-268 (1982)] in strain JM83 [Vieira et al., Gene 19, 259-268 (1982)] is described in Example 1.

For use with plasmid pWM111 [Mandecki et al., Gene (1986), in press] in strain JM83 where the distance mutation-cleavage site was 3 bases, the oligonucleotide sequence was from residue one to 59 or the non-coding strand of the C5a gene fragment [Mandecki et al., Proc. Natl. Acad. Sci. (USA), 82, 3543-3547 (1985)]. The mutation introduced was an A → G transition at position 24, which created a HaeIII site. For use with plasmid pWM111 where a 59-mer was employed and where the mutation to cleavage site distance was 0, the same oligonucleotide was used, except that the mutation introduced was a C → G transversion at position 27, which created a HpaII site.

For use with plasmid pWM111 where a 60-mer was employed, the oligonucleotide sequence corresponded to residues 3819-3878 of the coding strand of the $\beta$-lactamase gene of pBR322 [Sutcliffe, Cold Spring Harbor Symp. Quant. Biol., 43, 77-90 (1978)]. The mutation, a G → A transition at position 3849, destroyed the ScaI and RsaI sites.

The following E. coli strains were employed as host strains in these experiments and in the preceding Examples: CSH26 [ (lac pro) ara thi] [Miller, "Experiments in Molecular Genetics," Cold Spring Harbor Laboratory, Cold Spring Harbor, NY, p. 17 (1972)], JM832 [ara, (lac proAB), o80, lacZ M15] Vieira et al., Gene 19, 259-268 (1982) and MC1009 [ (lacIPOZY)X74, rpsL, galK, galU, (ara, leu)7697)] [Casadaban et al., J. Mol. Biol. 138, 179-207 (1980)]. Plasmids pUC9 [Vieira et al., Gene 19 259-268 (1982)] and pN01523 [Dean, Gene 15, 99-102 (1981)] were obtained from Pharmacia, Piscataway, New Jersey, and pWM111 was from the collection of Abbott Laboratories, Abbott Park, Illinois.

## Example 10

The procedure of Example 1 may be modified for the purpose of introducing defined mutations into chromosomes in situ.

Cells may be treated in order to induce double-strand breaks through the use of radiation [e.g. according to the procedure of Hutchinson et al., Prog. Nucl. Acid. Res., 32, 115-154 (1985)], or through the use of drugs [e.g. according to the procedure of Walker et al., Ann. Rev. Genet., 19, 103-126 (1985)], or through the use of derivatized DNA probes [e.g., according to the procedures of Dreyer et al., Proc. Natl. Acad. Sci (USA), 82, 968-972 (1985) and Dervan, Science, 232, 464-471 (1986)]. Oligonucleotides embodying the desired mutation are introduced into the cells, e.g. by $CaCl_2$-mediated transformation as described in Example 1 or electroporation as described in Smithies et al., supra. The cells are maintained under conditions which permit their viability and do not interfere with DNA repair within the cells. The cells are then screened for the presence of the desired mutation [e.g. by Southern blot analysis as set forth in Williams et al., Nature, 310, 476-480 (1984)] after a period of time sufficient to permit DNA repair to occur.

## Example 11

Especially in the case where derivatized oligonucleotide probes are employed to target the introduction of double-strand breaks to selected locations in a chromosome, the procedure according to Example 10 may be employed for gene therapy (i.e. for genetic modification leading to a desired alteration in phenotype.

Specifically, upon identification of a sequence in a portion of a chromosome which may be altered to produce a desired phenotype in a cell or an organism, oligonucleotide probes may be constructed which embody the mutation required to produce the desired phenotype. Cells (including but not limited to egg cells) may be extracted from an organism, or cells of a cell line may be used. A double-strand break may be created in a chromosome within the cells by the use of derivatized DNA probes specific for the site to be mutated according to the procedure of Dreyer et al., supra, or Dervan, supra. More than one strand break may be induced where, for example, a segment of a chromosome :s to be deleted or replaced. Oligonucleotides including the desired mutat:on may then be introduced into the cells by a modification of the procedure of Example 1 or by a number of techniques such as microinjection [see e.g. Brinster et al., Proc. Natl. Acad. Sci (USA), 82, 4438-4442 (1985)], electroporation [see e.g. Smithies et al., supra], or fusion with a liposome containing the oligonucleotides [see e.g. Lurquin, Nucleic Acids Res., 6, 3773-3784 (1979)]. The cells may then be screened for the presence of the desired mutation and introduced into an organism [see e.g. Williams et al., supra] or cultured as appropriate.

## Example 12

The present invention may be applied to DNA clone a DNA segment by covalently joining the DNA segment to a linearized vector by using two oligonucleotides which respectively span a break between a first end of the DNA to be cloned and a first end of a linearized vector, and a second end of DNA to be cloned and a second end of a linearized vector.

Example 13

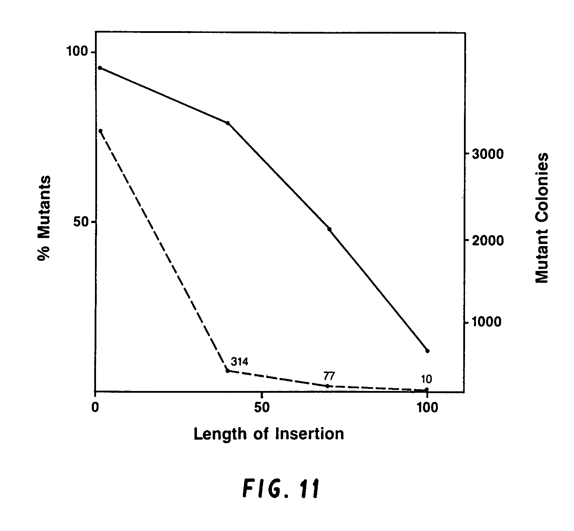

Bridge mutagenesis is an efficient technique to generate insertions in the vicinity of a unique restriction site. In this example, the feasibility of cloning relatively large inserts (up to 100 nt) through bridge mutagenesis is established. A set of oligonucleotides with inserts of different size was synthesized. The insert lengths were 1, 40, 70 and 100 nt. The lengths of oligonucleotides including the two arms providing homology to the plasmid were 31, 70, 100 and 130 nt, respectively. The plasmid pWM300 was used as the cloning vector. All of the oligonucleotides yielded plasmids with inserts (Fig. 11). However, the process becomes less efficient with increasing insert size, as indicated by the number of mutant colonies per plate (which dropped from 3900 colonies with 1 nt insert to 77 colonies with 70 nt insert), or by the percent of mutants among all colonies (dropped from 95% to 48%, respectively).

The results demonstrate that bridge mutagenesis is a useful, fast and simple method of cloning large DNA sequences. The technique became the basis for the presented gene synthesis method.

Example 14

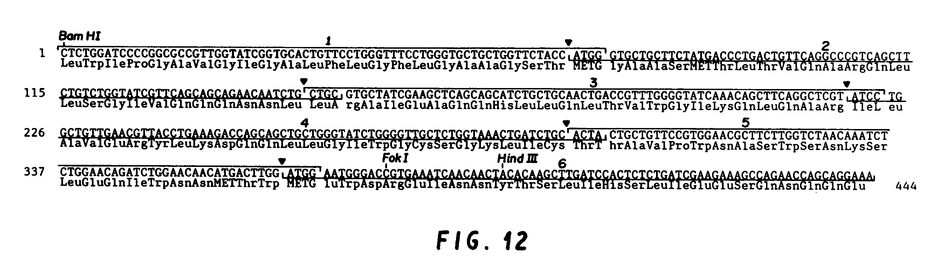

The oligonucleotide sequence shown in Figure 11 (which corresponds to amino acid residues 1 to 143 of the P41 protein of the human immunodeficiency virus (HIV)) was cloned into a plasmid vector pWM500 using the procedure of the present invention. The oligonucleotide of Figure 11 was synthesized using the phosphoramidite method. The oligonucleotide contained two "arms"extending from either end of the sequence of Figure 11, as shown in Figure 12.

Each of the two arms contains a sequence which corresponds to the DNA sequence on one side of a Sma I cleavage site on the pWM500 plasmid. The pWM500 plasmid can be substituted with many other plasmids, and the nucleotide sequences of the "arms" can be changed to correspond to the nucleotide sequences on either side of the double-strand DNA break. The restriction enzyme used to generate the said break should preferably generate blunt DNA ends. The plasmid pWM500 was cleaved with Sma I restriction endonuclease.

The oligonucleotide of Figure 12 (20 pmoles) and Sma I digested pWM500 (50 ng) were added to a denaturation buffer (10 mM Tris H-Cl, pH 8.0, 10 mM KCl, 5 mM MgCl$_2$), and heated for three minutes at 100°C (boiling water bath). The mixture was cooled for five minutes at room temperature. The mixture was added to competent JM83 cells (200 ul [ara, (lac-proAB), 80, lacZ DM15]) prepared by CaCl$_2$ treatment. The cellular mixture was incubated for five minutes on ice, and heat shocked at 37°C for three minutes. LB broth (2 mL) was added; and the mixture was incubated at 37°C for 60 minutes. The cell pellet was then spread on LB plates containing ampicillin 100 ug/ml). Cells carrying the appropriate plasmid were isolated with known techniques, and grown. DNA was then extracted from the cells, purified on a CsCl gradient, and the insert was sequenced.

Example 15

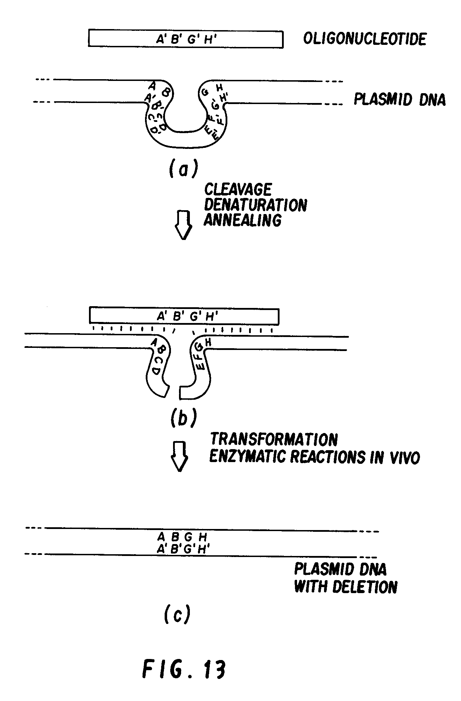

The process of this invention can also be used to delete DNA sequences from plasmids in the vicinity of a double stranded break (e.g., a Sma I break). The process of deleting DNA sequences is shown in Figure 13.

A deletion of a DNA sequence in plasmid DNA can be accomplished by synthesizing an oligonucleotide with a DNA sequence which corresponds to the DNA sequence on either side of the proposed deletion. For example, as shown in Fig. 13a, the oligonucleotide sequence is A'B'G'H', and the DNA sequence on either side of the uncut plasmid DNA is

```
A B C D E F G H         C D E F
A'B'C'D'E'F'G'H' where C'D'E'F'
```

is to be deleted. The plasmid is cleaved with an appropriate restriction enzyme which cuts within the sequence to be deleted. The mixture of oligonucleotide and plasmid DNA is denatured (as described in the previous examples), and cooled. This possibly allows the formation of a structure as shown in Fig. 13(b) where the oligonucleotide bridges and anneals to the corresponding sequences in the plasmid DNA on both sides of the break. Competent E. coli. cells are then transformed with the mixture. It has been found that a large portion of clones obtained will contain plasmids with the deletion expected, as shown in Fig. 13(c), i.e.

```
A  B  G  H
A'B'G'H'.
```

Example 16

Gene Synthesis by „Walking"

The present invention can also be used to insert plural DNA sequences into plasmid DNA, one insert at a time where the inserts form a continuous DNA region in the plasmid.

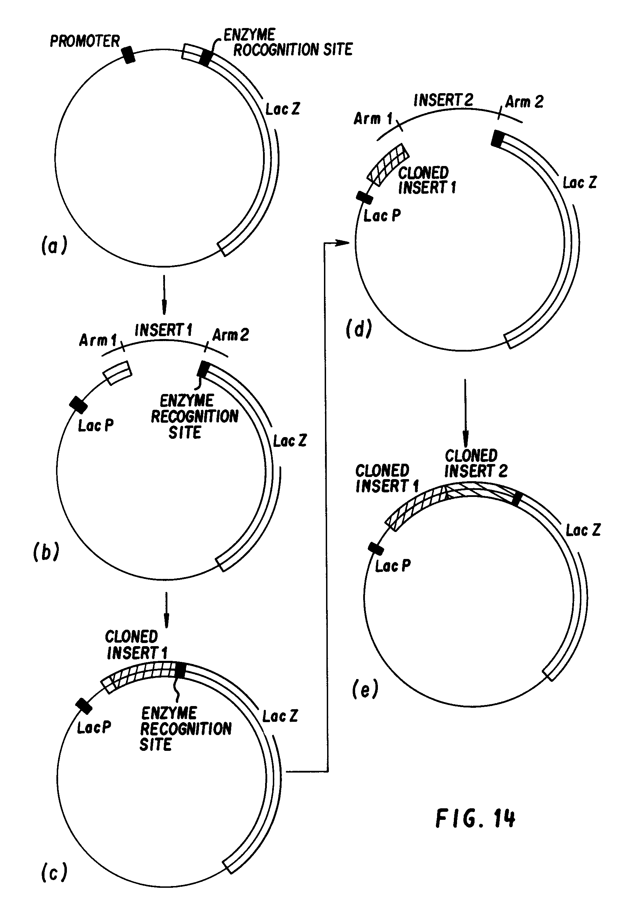

As shown in Fig. 14, a plasmid is chosen or constructed having a restriction enzyme recognition site for an enzyme which cleaves outside of the recognition site, for example Bsm I or other Class IIS restriction enzyme. Preferably, the restriction site is between a promoter and an indicator gene sequences, e.g. a lacP promoter and a lacZ indicator gene fragment. The promoter and indicator gene sequences are for screening and expression. The inserts are cloned sequentially. First, the plasmid is cleaved adjacent the Bsm I site with Bsm I. A oligonucleotide sequence having three regions is prepared. The first region ("arm 1") corresponds to the DNA sequence on one side of the break. The second region ("arm 2") corresponds to the DNA sequence on the other side of the break. The third region is the insert, and is located between the first and second regions. The oligonucleotide is mixed with the cleaved plasmid, and denatured (as described in the previous examples). The mixture is introduced into competent E. coli. cells through transformation. The cells are screened for the presence of the insert using the expression of the lacZ gene fragment.

The plasmid DNA is initially selected or constructed so that the lacZ gene fragment is in-frame (Fig. 14-(a)). When the insert is designed and cloned properly, the lacZ will be out-of-frame, so the $\beta$-galactosidase fragment will not be expressed. Thus, the colonies with the proper inserts will be white.

The procedure can be repeated with a second insert ("insert 2"; Fig. 14d). Examples of gene synthesis by walking are provided in Examples 17-24 where synthesis of expression vectors for the third factor of the human anaphylatoxin complement system, C3a, is described.

Example 17

Human Anaphylatoxin Complement Gene Synthesis

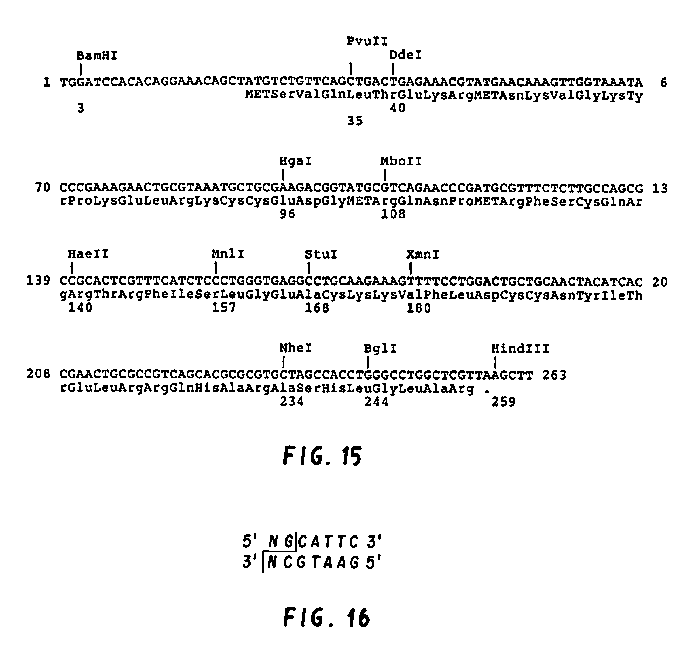

The third factor of the human anaphylatoxin complement system, C3a, was used as a model gene to synthesize using gene synthesis by „walking" as described in Example 16. Human C3a is a fairly small, highly cationic protein with a molecular weight of approximately 9,000 daltons (77 amino acids). Fig. 15 shows the amino acid sequence. A nucleic acid sequence (Fig. 15) was derived from the amino acid data by generating a sequence which contained as many unique restriction sites as possible in addition to using E. coli. codon preference for highly expressed genes (Gouy, M., et al., Nucleic Acids Res., 10[22], 7055, 1982). Unique restriction sites were required for future mutagenesis by fragment replacement.

The complete derived nucleotide sequence for the C3a gene is shown in Fig. 15. It consists of unique BamHI/HindIII ends at the 5$'$ and 3$'$ ends, respectively. These sites were added for cloning into an expression vector (e.g. pWM200) after the gene was synthesized. There is a lac ribosome binding site between the BamHI site and the initiator Met codon. The entire C3a sequence follows with a single TAA translational stop codon which constitutes part of the terminal HindIII site.

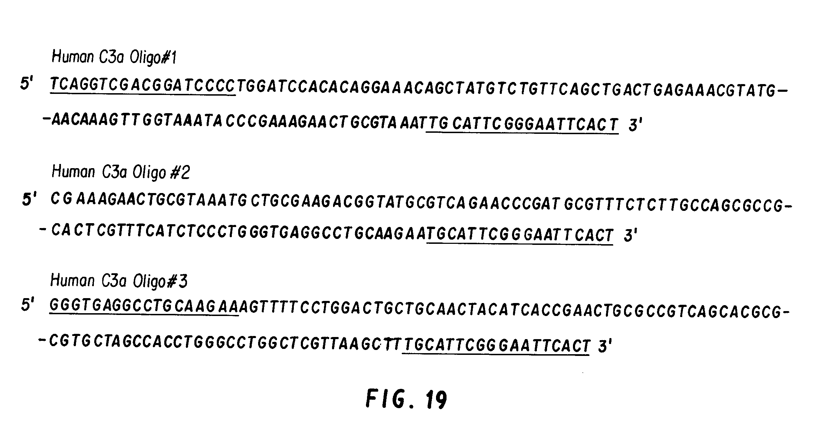

The gene was subdivided into three synthetic oligodeoxyribonucleotides (Fig. 19), each of which contained a sequential portion of the whole gene. Oligo #1 (125mer) consisted of 18 nt on both 5$'$ and 3$'$ ends which were complementary to the sequence on both sides of the BsmI cleavage site of pMH1 after cutting with BsmI. The 3$'$ "arm" included the BsmI sequence for regeneration of the site at the 3$'$ region of the gene. The 89 base insert of oligo #1 contained the BamHI sequence, ribosome binding site, initiator Met codon, and the first 21 amino acid residues representing the amino terminal region of C3a. Oligo #2 (124mer) contained 18 nt on the 5$'$ end which were complementary to the last 18 nt of the gene sequence from oligo #1; 18 nt on the 3$'$ end complementary to the vector sequence and including the BsmI site; and an 88 base insert containing codons coding for the amino acids 22-50 of C3a. Oligo #3 (122mer) included 18 nt on the 5' arm which were complementary to the last 18 nt of the gene sequence from oligo #2; 18 nt on the 3' end complementary to the vector sequence and including the BsmI site; and an 86 base insert containing the codons coding for the last 22 amino acids of C3a with a single TAA stop codon and a terminal HindIII site.

The final plasmid constructs containing the first third, the first two-thirds, and the complete C3a gene were designated as pC3H1, pC3H2, and pC3H3, respectively. The synthesis of these three plasmids is described in Examples 20, 21 and 22, respectively.

All synthetic oligodeoxyribonucleotides described here were synthesized on an Applied Biosystems 380A Synthesizer using nucleoside 0-(2-cyanoethyl)-N,N-diisopropylphosphoramidites. Synthesis was done on 0.2 umole 1000 A controlled pore glass columns (Applied Biosystems) using the Small Scale Cycle recommended by ABI.

Oligodeoxyribonucleotides were purified on 10% polyacrylamide (1/20 bis-acrylamide), 1X TBE (89 mM Tris-borate, 89 mM boric acid, 2mM EDTA) gel under denaturing conditions. Product DNA bands were cut out of the gel and eluted overnight at 37°C by soaking in 1.0 ml Elution Buffer (0.5 M ammonium acetate, 1mM EDTA). The DNA was ethanol precipitated twice and resuspended in 50 ul dH$_2$O.

Acrylamide, bis-acrylamide, Tris and boric acid were purchased from International Biotechnologies, Inc. EDTA was from Sigma, ammonium acetate was from Fisher. All chemicals used for DNA synthesis were purchased from Applied Biosystems.

Example 18

Construction of Cloning Vectors

A parent cloning vector was designed to include a unique restriction site within the SmaI site of the multicloning sequence of the pUC derived series of plasmids. The only requirement of the unique restriction site was that it produces a single double-strand cut with at least one of the strands being cut at the junction of the site. BsmI fulfills this requirement. It has a 6-base nonpalindromic recognition sequence and produces a double-strand cut with a 2-base 3' protruding end. It is isolated from Bacillus stearothermophilus NUB 36 and sold through New England BioLabs. Figure 16 shows the BsmI recognition sequence and cutting pattern, where N represents any nucleotide.

The pUC series of plasmids represent a base from which to construct the parent cloning vector of the present invention. These plasmids contain the lacZ gene with a multicloning site in the amino terminus region of the β-galactosidase in addition to a β-lactamase gene and a ColEI-type origin of replication. It has a high copy number, and can be amplified with chloramphenicol. Another advantage is the absence of any BsmI sites on the plasmid.

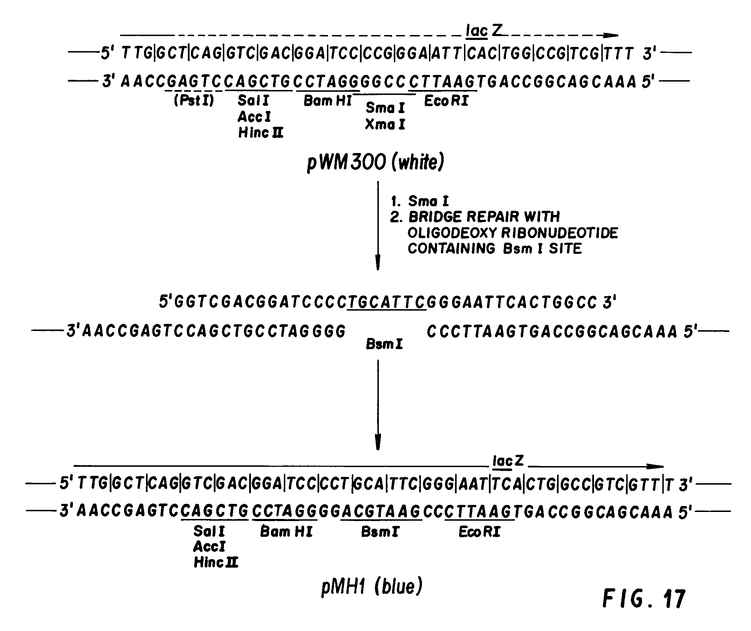

The construction of pMH1 from pWM300 is shown in Figure 17. pWM300 is a derivative of pUC9 (available from Pharmacia) with a single base deletion within the PstI site of the multicloning site. This deletion causes a frameshift mutation in the lacZ and results in white colonies on an X-Gal plate.

A BsmI site was inserted into the SmaI site of pWM300 by means of a 37mer synthetic oligodeoxyribonucleotide using the protocol for oligonucleotide-directed double-strand break repair of this invention. The total length of the insertion was a 7mer which caused a frameshift of the lacZ back into frame and resulted in blue recombinant colonies.

50 ng SmaI cut pWM300 plus 20 picomoles of the synthetic 37mer was transformed into 200 ul frozen competent E. coli. JM83 cells (Vieira, J. et al.,Gene, 19 259, 1982) and plated on LB plates (GIBCO) containing 100 ug ampicillin and 1.6 mg X-Gal (5-bromo-4-chloro-3-indolyl β-D-galactoside).Plates were incubated for 15 hours at 37°C and scored for blue colonies. Of a total of 546 colonies, 528 were blue which represents a mutagenesis efficiency of approximately 97%. Two blue colonies were picked and grown in 200 ml LB broth (10 g Bacto-tryptone, 5 g Bacto-yeast extract, 10 g NaCl/l) plus 100 ug/ml ampicillin. The plasmid DNA was amplified using 20 ug/ml chloramphenicol (Frenkel, L., et al., DNA, 5[6], 539, 1986] and isolated using the NaOH/SDS lysis procedure [Birnboim, H., et al., Nucleic Acids Res., 7:1513, 1979).

To check for the presence of the BsmI site, 500 ng of plasmid DNA was cut with 4 units BsmI under conditions recommended by New England BioLabs. The BsmI treated DNA was run on a 0.8% agarose (Sigma), 0.5X TBE (45 mM Tris-borate, 45 mM boric acid, 1 mM EDTA) gel along uncut DNA. Both plasmid preps showed a single band with the correct MW. This plasmid was named pMH1 and was used for all subsequent cloning.

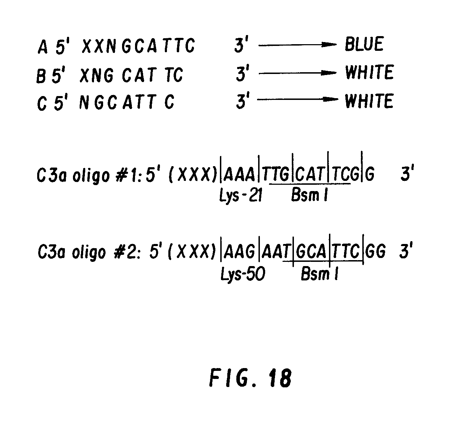

Fig. 18 shows how the alternating X-Gal color selection works where X and N in Fig. 18 represent any oligonucleotide. By designing the inserted oligodeoxyribonucleotide reading frame to be in or out of frame with the lacZ, the color is alternated between blue or white. Fig. 18 also shows how the three C3a oligodeoxyribonucleotides were designed to utilize this color selection scheme. Oligo #1 produced a white phenotype by causing a frameshift in the lacZ with an insertion of a T following Lys-21. This inserted T is the first base of the Cys-22 codon. Oligo #2 caused a white-to-blue shift by inserting the first two A residues of the Lys-51 codon. Oligo #3 produced a blue-to-white change by virtue of a TAA translational stop codon.

Example 19

Cutting Cloning Vector with Bsm I

Prior to each sequential cloning step in the present invention the vector carrying the recombinant gene sequence must be cut with BsmI. Several variations of this step have been worked out in order to accommodate the particular needs of the investigator.

The usual protocol calls for cutting 500 ng of CsCl purified plasmid DNA with 8 units of BsmI in a total reaction volume of 50 ul to give a final DNA concentration of 10 ng/ul. The digestion is carried out 3-4 hours at 65°C in a 200 ul flame sealed Accupette glass pipet. Approximately 50 ng (5 ul) is used directly for mutagenesis with 20 picomoles of the appropriate oligodeoxyribonucleotide.

If high background is a problem, the BsmI cut vector may be purified from uncut DNA by PAGE. This protocol requires a larger amount of DNA for cutting because of losses from the gel. Approximately 5 ug of plasmid DNA is digested with 12 units of BsmI in a total reaction volume of 50 ul for 5 hours at 65°C. The sample is loaded onto a 5% polyacrylamide (1/50 bisacrylamide), 1X TBE (89 mM Tris-borate, 89 mM boric acid 2 mM EDTA) and electrophoresed for 2 hours at 10 watts. Under these conditions, cut vector DNA migrates at a faster rate compared to uncut supercoiled DNA. The gel is soaked for a few minutes in dH$_2$O containing 0.5 ug/ml ethidium bromide and the DNA is visualized by UV illumination. The band is cut out and crushed in an eppendorf tube. The DNA is eluted by soaking the crushed acrylamide gel slice in 0.5 ml Elution Buffer (0.5 mM ammonium acetate, 1 mM EDTA) overnight at 37°C.

The acrylamide is filtered away from the buffer solution by centrifugation through a 0.2 um Centrex Microfilter tube (Schleicher & Schuell). The DNA is precipitated by adding two volumes ethanol and storing at -20°C for 2-3 hours. The DNA is microfuged and resuspended in 10 ul sterile dH$_2$O.

The DNA concentration may be approximated by running 1 ul on an 0.8% agarose, 0.5X TBE gel with DNA reference standards. A typical run yields 20% from the original 5 ug to give a final concentration of approximately 50 ng/ul.

If a large number of gene fragments are used for synthesis of a gene using the present invention and time is a limiting factor, isolation and CsCl purification of plasmid DNA can be eliminated. Mini-prep DNA may be substituted for CsCl DNA. 500 ng mini-prep DNA may be cut with 8 units of BsmI at 65°C for 3-4 hours and used directly for mutagenesis without any phenol/chloroform extractions or RNAse treatment. Although this particular protocol is rapid, high background can be a problem.

Example 20

Construction of pC3H1 from pMH1

pMH1 was cut with BsmI using conditions described in Example 19. The linearized vector was mixed and annealed with C3a oligonucleotide #1 and transformed into frozen competent JM83 cells using the protocol for oligonucleotide-directed double-strand break repair described in this invention. Several transformants which exhibited a white phenotype were checked for the C3a gene fragment by digesting the recombinant plasmid with PvuII. Mutants carrying the C3a fragment were identified by displaying the proper PvuII restriction pattern. Plasmid DNA from several mutants were purified by CsCl gradient and sequenced. The recombinant plasmid containing the first gene fragment of human C3a was designated as pC3H1.

Example 21

Construction of pC3H2 from pC3H1

pC3H1 was cut with BsmI using conditions described in Example 19. The linearized vector was mixed and annealed with C3a oligonucleotide #2 and transformed into frozen competent JM83 cells using the protocol for oligonucleotide-directed double-strand break repair described in this invention. Several transfor-

EP 0 319 759 B1

mants which exhibited a blue phenotype were checked for the first two C3a gene fragments by digesting the recombinant plasmid with PvuII. Mutants carrying the first two C3a fragments were identified by displaying the proper PvuII restriction pattern. Plasmid DNA from two mutants was purified by CsCl gradient and sequenced. The recombinant plasmid containing the first two-thirds of the human C3a gene was designated as pC3H2.

Example 22

Construction of pC3H3 from pC3H2

pC3H2 was cut with BsmI using conditions described in Example 19. The linearized vector was mixed and annealed with C3a oligonucleotide #3 and transformed into frozen competent JM83 cells using the protocol for oligonucleotide-directed double-strand break repair described in this invention. Several transformants which exhibited a white phenotype were checked for the complete C3a gene by digesting the recombinant plasmid with PvuII. Mutants carrying the complete C3a gene were identified by displaying the proper PvuII restriction pattern. Plasmid DNA from a single mutant was purified by CsCl gradient and sequenced. The recombinant plasmid containing the complete human C3a gene was designated as pC3H3.

Example 23

Transformation of E.Coli. with pC3H3

Transformation was the same as described in Example 1.

Numerous modifications of and variations in the present invention are expected to occur to those skilled in the art upon consideration of the foregoing specification. For example, although the term oligonucleotide is employed herein, it will be obvious to one skilled in the art to employ any linear, single-stranded deoxyribonucleic acid in the methods according to the present invention. Likewise, it will be readily apparent to one skilled in the art that the present invention may be employed to insert, and delete and substitute (i.e., simultaneously delete and insert) groups of nucleotides constituting one or more codons in order to obtain genes encoding analogs of polypeptides from a gene encoding a naturally occurring form.

Consequently, it is intended that the invention be limited only to the extent reflected by the appended claims.

**Claims**

1.  A method for sequential gene assembly, comprising:
    a) providing a DNA molecule with a restriction enzyme recognition site for a restriction enzyme which cleaves outside the recognition site;
    b) cleaving the DNA molecule with said enzyme;
    c) providing a first oligonucleotide having a first portion complementary to a nucleotide seguence in said DNA molecule on one side of the cleavage site, a second portion complementary to a said DNA molecule nucleotide sequence on the other side of said cleavage site, and a third portion between the first and second portions which has a nucleotide sequence containing a portion of a gene sequence to be inserted into said DNA molecule;
    d) exposing said cleaved DNA molecule to said first oligonucleotide, and denaturing the DNA molecule;
    e) transforming a host cell with said first oligonucleotide and said DNA molecule to insert said fragment into the DNA molecule;
    f) repeating steps b - e with at least one other oligonucleotide having a third portion with a nucleotide sequence complementary to another fragment to be inserted.

2.  The method of claim 1 wherein the majority of a lacZ coding region is 3' to said restriction enzyme site.

3.  The method of claim 2 further including screening said DNA molecule for lacZ expression for proper insertion of said inserts.

4.  The method of claim 2 wherein said restriction enzyme site is 3' to a promoter.

17

**5.** The method of claim 4 wherein said promoter is lacP.

**6.** The method of claim 2 wherein said inserts comprise a gene sequence for human C3a.

**Patentansprüche**

**1.** Ein Verfahren zum sequentiellen Genzusammenbau umfassend:
a) Bereitstellen eines DNA-Moleküls mit einer Restriktionsenzym-Erkennungssequenz für ein Restriktionsenzym, welches außerhalb der Erkennungsstelle schneidet,
b) Schneiden des DNA-Moleküls mit dem besagten Enzym,
c) Bereitstellen eines ersten Oligonukleotids, das eine erste Teilsequenz aufweist, die zu einer Nukleotidseqenz des besagten DNA-Moleküls auf einer Seite der Schnittstelle komplementär ist, weiter eine zweite Teilsequenz, die zu einer Nukleotidsequenz des genannten DNA-Moleküls auf der anderen Seite der Schnittstelle komplementär ist, und eine dritte Teilsequenz zwischen der ersten und zweiten Teilsequenz enthält, die eine Nukleotidsequenz aufweist, die einen Anteil der Gensequenz beinhaltet, die in das besagte DNA-Molekül eingefügt werden soll,
d) Exponieren des besagten geschnittenen DNA-Moleküls mit dem ersten ersten Oligonukleotid, und Denaturieren des DNA-Moleküls,
e) Transformieren einer Wirtszelle mit dem besagten ersten Oligonukleotid und dem besagten DNA-Molekül, um das Fragment in das DNA-Molekül einzufügen,
f) Wiederholen der Stufen b - e mit mindestens einem anderen Oligonukleotid, das eine dritte Teilseqenz mit einer Nukleotidsequenz enthält, die zu einem anderen zu insertierenden Fragment komplementär ist.

**2.** Verfahren gemäß Anspruch 1, worin der größere Teil einer lacZ kodierenden Region sich in 3'-Richtung zu besagter Restriktionsenzym-stelle befindet.

**3.** Verfahren gemäß Anspruch 2, das zudem ein Screening des besagten DNA-Moleküls für die lacZ-Expression zur richtigen Insertion der genannten Inserts einschließt.

**4.** Verfahren gemäß Anspruch 2, worin die besagte Restriktionsenzym-stelle sich in 3'-Richtung zu einem Promotor befindet.

**5.** Verfahren gemäß Anspruch 4, worin der besagte Promotor lacP ist.

**6.** Verfahren gemäß Anspruch 2, worin die besagten Inserte eine Gensequenz für humanes C3a umfassen.

**Revendications**

**1.** Procédé d'assemblage séquentiel de gène consistant à :
a) se pourvoir d'une molécule d'ADN ayant un site de reconnaissance d'une enzyme de restriction pour une enzyme de restriction qui clive à l'extérieur du site de reconnaissance ;
b) cliver la molécule d'ADN avec ladite enzyme ;
c) se pourvoir d'un premier oligonucléotide ayant une première portion complémentaire d'une séquence nucléotidique dans ladite molécule d'ADN d'un côté du site de clivage, une seconde portion complémentaire d'une séquence nucléotidique de ladite molécule d'ADN de l'autre côté dudit site de clivage et une troisième portion entre la première et la seconde portion qui a une séquence nucléotidique contenant une portion d'une séquence du gène à insérer dans ladite molécule d'ADN ;
d) exposer ladite molécule d'ADN clivée audit premier oligonucléotide et dénaturer la molécule d'ADN ;
e) transformer une cellule hôte avec ledit premier oligonucléotide et ladite molécule d'ADN pour insérer ledit fragment dans la molécule d'ADN ;
répéter les étapes b-e avec au moins un autre oligonucléotide ayant une troisième portion avec une séquence nucléotidique complémentaire d'un autre fragment à insérer.

**2.** Procédé selon la revendication 1, dans lequel la majorité d'une région codante lacZ est en 3' dudit site

18

de l'enzyme de restriction.

3. Procédé selon la revendication 2, comprenant de plus le criblage de ladite molécule d'ADN pour l'expression de lacZ pour l'insertion appropriée desdits inserts.

4. Procédé selon la revendication 2, dans lequel ledit site de l'enzyme de restriction est en 3' d'un promoteur.

5. Procédé selon la revendication 4, dans lequel ledit promoteur est lacP.

6. Procédé selon la revendication 2, dans lequel lesdits inserts comprennent une séquence du gène du C3a humain.

EP 0 319 759 B1

Translation
Start

|       | -40 | -30 | -20 | -10 | 1 | 10 | 20 |
|-------|-----|-----|-----|-----|---|----|----|

pUC9  ATGACCATGATTACGCCAAGCTTGGCTGCAGGTCGACGGATCCCCGGGAATTCACTGGCCGTCGTTTTACAACGT

HindIII    PstI    SalI  BamHI SmaI EcoRI
                     AccI       Xmal
                     HincII

|       | -40 | -30 | -20 | -10 | 1 | 10 | 20 |
|-------|-----|-----|-----|-----|---|----|----|

pWM300  ATGACCATGATTACGCCAAGCTTGGCT-CAGGTCGACGGATCCCCGGGAATTCACTGGCCGTCGTTTTACAACGT

DdeI

White
Colonies | Mutagenesis

Blue
Colonies

|       | -40 | -30 | -20 | -10 | 1 | 10 | 20 |
|-------|-----|-----|-----|-----|---|----|----|

pWM301  ATGACCATGATTACGCCAAGCTTGGCT-CAGGTCGACGGATCCCCAGGGAATTCACTGGCCGTCGTTTTACAACGT

DdeI                          BstNI

## FIG. 1

Oligonucleotide
5'
CGGATCCCC A GGGAATTCAC

Linearized
Plasmid DNA

5'
CGGATCCCC   GGGAATTCAC
GCCTAGGGG   CCCTTAAGTG

↑
Cleavage Site

**pWM300**

CGGATCCCC A GGGAATTCAC
CCCTAGGGG T CCCTTAAGTG

↑
Mutation

**pWM301**

## FIG. 2

5' CCAAGCTTGGCTCAGGTCGACGGATCCCC**A**GGGAATTCACTGGCCGTCGTTTTACAACGT 60

pWM300

*FIG. 3*

*FIG. 4*

FIG. 5

FIG. 6

```
        5'
Mbol ──── T ──────────────────────────────────────── 40
Nsil ────────── C ──────────────────────────────────── 35
Mnll ──────────────── TC ──────────────────────────── 26
Pstl ──────────────────────── G ──────────────────── 17
Hpall ──────────────────────────────── C ──────────── 8
Mnll ──────────────────────────────────────── T ──── 2
BstNI ─────────────────────────────────────────── A ──── 0
  5' ···ATGA'CCATG'ATTACGCC'AAGCTTGGCT'CAGGTCGAC'GGATCC'CC   GGGAATTCACTGGCC···
pWM300                                                ↑
```

**FIG. 7**

**FIG. 8**

EP 0 319 759 B1

5′

5′···CCAAGCTTGGCTCAGGTCGACGGATCCCCGGGAATTCACTGGCCGTCGTTTTACAACGT···

pWM300

HindIII

SalI
AccI

BamHI

XmaI
SmaI

EcoRI

**FIG. 9**

Distance from Cleavage Site (nt)

**FIG. 10**

**FIG. 11**

*Bam HI*

1

▼

2

```
CTCTGGATCCCCGGCGCCGTTGGTATCGGTGCACTGTTCCTGGGTTTCCTGGGTGCTGCTGGTTCTACC ATGG GTGCTGCTTCTATGACCCTGACTGTTCAGGCCCGTCAGCTT
LeuTrpIleProGlyAlaValGlyIleGlyAlaLeuPheLeuGlyPheLeuGlyAlaAlaGlySerThr METG lyAlaAlaSerMETThrLeuThrValGlnAlaArgGlnLeu
```

▼

3

```
CTGTCTGGTATCGTTCAGCAGCAGAACAATCTG CTGC GTGCTATCGAAGCTCAGCAGCATCTGCTGCAACTGACCGTTTGGGGTATCAAACAGCTTCAGGCTCGT ATCC TG
LeuSerGlyIleValGlnGlnGlnAsnAsnLeu LeuA rgAlaIleGluAlaGlnGlnHisLeuLeuGlnLeuThrValTrpGlyIleLysGlnLeuGlnAlaArg IleL eu
```

4

▼

5

```
GCTGTTGAACGTTACCTGAAAGACCAGCAGCTGCTGGGTATCTGGGGTTGCTCTGGTAAACTGATCTGC ACTA CTGCTGTTCCGTGGAACGCTTCTTGGTCTAACAAATCT
AlaValGluArgTyrLeuLysAspGlnGlnLeuLeuGlyIleTrpGlyCysSerGlyLysLeuIleCys ThrT hrAlaValProTrpAsnAlaSerTrpSerAsnLysSer
```

▼

*Fok I*          *Hind III*     6

```
CTGGAACAGATCTGGAACAACATGACTTGG ATGG AATGGGACCGTGAAATCAACAACTACACAAGCTTGATCCACTCTCTGATCGAAGAAAGCCAGAACCAGCAGGAAA
LeuGluGlnIleTrpAsnAsnMETThrTrp METG luTrpAspArgGluIleAsnAsnTyrThrSerLeuIleHisSerLeuIleGluGluSerGlnAsnGlnGlnGlu  444
```

# FIG. 12

A' B' G' H'     OLIGONUCLEOTIDE

PLASMID DNA

(a)

CLEAVAGE
DENATURATION
ANNEALING

A' B' G' H'

(b)

TRANSFORMATION
ENZYMATIC REACTIONS IN VIVO

A B G H
A'B'G'H'

PLASMID DNA
WITH DELETION

(c)

FIG. 13

29

FIG. 14

```
                                              PvuII
            BamHI                              |    DdeI
            |                                  |    |
  1 TGGATCCACACAGGAAACAGCTATGTCTGTTCAGCTGACTGAGAAACGTATGAACAAAGTTGGTAAATA  6
                             METSerValGlnLeuThrGluLysArgMETAsnLysValGlyLysTy
            3                                       40
                                            35


                              HgaI              MboII
                              |                 |
 70 CCCGAAAGAACTGCGTAAATGCTGCGAAGACGGTATGCGTCAGAACCCGATGCGTTTCTCTTGCCAGCG 13
    rProLysGluLeuArgLysCysCysGluAspGlyMETArgGlnAsnProMETArgPheSerCysGlnAr
                              96              108


    HaeII               MnlI           StuI           XmnI
    |                   |              |              |
139 CCGCACTCGTTTCATCTCCCTGGGTGAGGCCTGCAAGAAAGTTTTCCTGGACTGCTGCAACTACATCAC 20
    gArgThrArgPheIleSerLeuGlyGluAlaCysLysLysValPheLeuAspCysCysAsnTyrIleTh
    140                 157            168            180


                         NheI           BglI           HindIII
                         |              |              |
208 CGAACTGCGCCGTCAGCACGCGCGTGCTAGCCACCTGGGCCTGGCTCGTTAAGCTT 263
    rGluLeuArgArgGlnHisAlaArgAlaSerHisLeuGlyLeuAlaArg .
                         234            244            259
```

# FIG. 15

```
5'  N G|C A T T C 3'
3' |N C G T A A G 5'
```

# FIG. 16

31

EP 0 319 759 B1

lac Z →

—5' TTG|GCT|CAG|GTC|GAC|GGA|TCC|CCG|GGA|ATT|CAC|TGG|CCG|TCG|TTT 3' —

— 3' AACCGAGTCCAGCTGCCTAGGGGCCCTTAAGTGACCGGCAGCAAA 5' —

(PstI)    Sal I     Bam HI  Sma I   EcoRI
           Acc I          Xma I
           Hinc II

**pWM300 (white)**

1. Sma I
2. BRIDGE REPAIR WITH
   OLIGODEOXY RIBONUDEOTIDE
   CONTAINING Bsm I SITE

5'GGTCGACGGATCCCCTGCATTCGGGAATTCACTGGCC 3'

—3'AACCGAGTCCAGCTGCCTAGGGG        CCCTTAAGTGACCGGCAGCAAA 5'—

                              BsmI

lac Z →

— 5' TTG|GCT|CAG|GTC|GAC|GGA|TCC|CCT|GCA|TTC|GGG|AAT|TCA|CTG|GCC|GTC|GTT|T 3' —

— 3' AACCGAGTCCAGCTGCCTAGGGGACGTAAGCCCTTAAGTGACCGGCAGCAAA 5' —

        Sal I     Bam HI    BsmI     EcoRI
        Acc I
        Hinc II

**pMH1 (blue)**

**FIG. 17**

A 5' XXNGCATTC 3' ⟶ BLUE
B 5' XNG CAT TC 3' ⟶ WHITE
C 5' NGCATT C 3' ⟶ WHITE

C3a oligo #1: 5' (XXX)|AAA|TTG|CAT|TCG|G 3'
Lys-21 Bsm I

C3a oligo #2: 5' (XXX)|AAG|AAT|GCA|TTC|GG 3'
Lys-50 Bsm I

FIG. 18

Human C3a Oligo#1

5' <u>TCAGGTCGACGGATCCCC</u>TGGATCCACACAGGAAACAGCTATGTCTGTTCAGCTGACTGAGAAACGTATG-

-AACAAAGTTGGTAAATACCCGAAAGAACTGCGTAAAT<u>TGCATTCGGGAATTCACT</u> 3'

Human C3a Oligo #2

5' CGAAAGAACTGCGTAAATGCTGCGAAGACGGTATGCGTCAGAACCCGATGCGTTTCTCTTGCCAGCGCCG-

-CACTCGTTTCATCTCCCTGGGTGAGGCCTGCAAGAA<u>TGCATTCGGGAATTCACT</u> 3'

Human C3a Oligo#3

5' <u>GGGTGAGGCCTGCAAGAA</u>AGTTTTCCTGGACTGCTGCAACTACATCACCGAACTGCGCCGTCAGCACGCG-

-CGTGCTAGCCACCTGGGCCTGGCTCGTTAAGCTT<u>TGCATTCGGGAATTCACT</u> 3'

## FIG. 19

EP 0 319 759 B1